# EUROPEAN PATENT APPLICATION

(11) **EP 1 325 711 A2**
(43) Date of publication of application: **09.07.2003**
(21) Application number: 02258852.9
(22) Date of filing: 23.12.2002
(51) Int. Cl.: A61B 17/86

(54) **Cannulated screw and associated driver system**

(30) Priority: 27.12.2001 US 32833
(71) Applicant: ETHICON, INC., Somerville, NJ 08876 (US)
(72) Inventor: Yuan, J. Jenny, Neshanic Station, New Jersey 08853 (US); Poandl, Thomas M., Metuchen, New Jersey 08840 (US)
(74) Representative: Mercer, Christopher Paul

(57) **Abstract**

A driver (10) for inserting cannulated screws (30) has a tool portion in the form of a hex wrench that slips into the cannula (38) of the screw (30). A tapered threaded tip (18) on the tool (10) extends beyond the front tip of the screw (30). An abutment surface (20) provided, e.g., by the toolholder (14) acts as a limit to rearward motion of the screw (30) when it is being screwed into a substrate such as bone. The threaded tip (18) of the tool (10) cuts the bone to allow the threads (32) of the cannulated screw (30) to follow into the substrate permitting use of a softer material, such as a bioabsorbable material for the screw (30).

## Description

### FIELD OF THE INVENTION

This invention relates to orthopedic screws and surgical procedures using same, and, more particularly, to interference screws for securing synthetic or biological tissue to bone.

### BACKGROUND OF THE INVENTION

The knee joint is one of the strongest joints in the body because of the powerful ligaments that bind the femur and tibia together. Notwithstanding, the knee is one of the most frequently injured joints, e.g., athletes frequently stress and tear knee ligaments. The large number of ligament injuries has given rise to numerous innovative surgical procedures and devices for replacing and reconstructing torn or dislocated ligaments, typically involving grafting autografts, allografts, or a synthetic construct, to the site of a torn or dislocated ligament. For example, the replacement of an anterior cruciate ligament (ACL) may involve transplanting a portion of the patellar tendon, looped together portions of semitendinosus-gracilis (hamstring) tendons, or donor achilles tendons, to attachment sites in the region of the knee joint.

The most widely used technique for the reconstruction of the ACL is known as the Jones procedure. The basic steps in the procedure include: harvesting a graft made from a portion of the patellar tendon with attached bone blocks; preparing the graft attachment site (e.g., drilling holes in opposing bones of the joint in which the graft will be placed); placing the graft in the graft attachment site; and rigidly fixing the bone blocks in place within the graft site, i.e., the holes or "bone tunnels". The screws used to fix the graft in place are called "interference screws" because they wedge between the bone block and the wall of the hole into which the bone block fits as they are screwed in. Typically, there is very little space between the bone block and the hole in the bone at the fixation site.

Interference screws are typically driven into the space between the bone block and the wall of the bone tunnel by placing the screw on a driver and then exerting a force on the driver and the screw in the direction of insertion while rotating the driver. The end of the driver may be received in a socket or groove located on the proximal end of the screw. More typically, the screw has a cannula or through hole in which the driver is inserted. The advantage of a cannulated screw is that the force used to drive the screw into the space between the bone block and the wall of the bone tunnel is spread over a larger area of the screw. This reduces the risk of damage to the screw or slippage of the driver relative to the screw as the screw is being driven into position.

Interference screws for anchoring ligaments to bone are typically fabricated from medically approved metallic materials that are not naturally absorbed by the body. A disadvantage of such screws is that once healing is complete, an additional surgical procedure may be required to remove the screw from the patient. Metallic screws may include a threaded shank joined to an enlarged head having a transverse slot or hexagonal socket formed therein to engage, respectively, a similarly configured, single blade or hexagonal rotatable driver for turning the screw into the bone. The enlarged heads on such screws can protrude from the bone tunnel and can cause chronic irritation and inflammation of surrounding body tissue.

Permanent metallic medical screws in movable joints can, in certain instances, cause abrading of ligaments during normal motion of the joint. Screws occasionally back out after insertion, protruding into surrounding tissue and causing discomfort. Furthermore, permanent metallic screws and fixation devices may shield the bone from beneficial stresses after healing. It has been shown that moderate periodic stress on bone tissue, such as the stress produced by exercise, helps to prevent decalcification of the bone. Under some conditions, the stress shielding which results from the long term use of metal bone fixation devices can lead to osteoporosis.

Biodegradable or bioabsorbable interference screws have been proposed to avoid the necessity of surgical removal after healing. Because the degradation of a biodegradable screw occurs over a period of time, support load is transferred gradually to the bone as it heals. This reduces potential stress shielding effects. Conventional bioabsorbable interference screws are softer and weaker than metallic compositions, such that they are not self-tapping, requiring the holes drilled into the bone to be tapped. The necessity to tap holes in the injured bone adds to the complexity of the surgical procedure and lengthens the time required to complete the operation.

Accordingly, there is a need for a device and method that would allow interference screws composed mainly of bioabsorbable materials to be inserted into bone where the tapping of the bone is concurrent with the insertion of the screw into the bone.

### SUMMARY OF THE INVENTION

The limitations of prior art apparatus for threading cannulated screws into a substrate are overcome by the present invention which includes a driver for threading a cannulated screw into a substrate. The driver has a tool with a screw engaging portion and a threaded tip. The screw engaging portion is extendable through a cannula formed in the cannulated screw and has a shape that substantially prevents rotation of the screw engaging portion relative to the cannulated screw when the screw engaging portion is extending through the cannula of the cannulated screw. The threaded tip extends beyond the screw when the screw engaging portion is inserted into the cannula of the cannulated screw.

### BRIEF DESCRIPTION OF THE FIGURES

Figure 1 is a perspective view of an exemplary embodiment of a driver and a cannulated interference screw that may be inserted into a substrate by the driver in accordance with the present invention.
Figure 2 is a side view of a driver and a cannulated interference screw in accordance with a second embodiment of the present invention with the interference screw in position on the driver.
Figure 3 is a partially cross-sectional view of the driver and interference screw of FIG. 2.

### DETAILED DESCRIPTION OF THE INVENTION

The present invention relates to orthopedic surgical drivers for screws for securing synthetic or biological connective tissue to a bone surface, such as, for example, attaching and maintaining a replacement anterior cruciate ligament (ACL) against a bone. The driver and method for using it would allow cannulated interference screws composed mainly of bioabsorbable materials to be inserted into bone where the tapping of the bone tunnel is concurrent with the insertion of the screw into the bone. The driver has means for tapping threads while it drives the screw into bone. The screw has threads to advance the screw into a position to establish an interference fit between the bone blocks and the bone tunnel, as well as, to hold the replacement anterior cruciate ligament (ACL) against the wall of the bone tunnel.

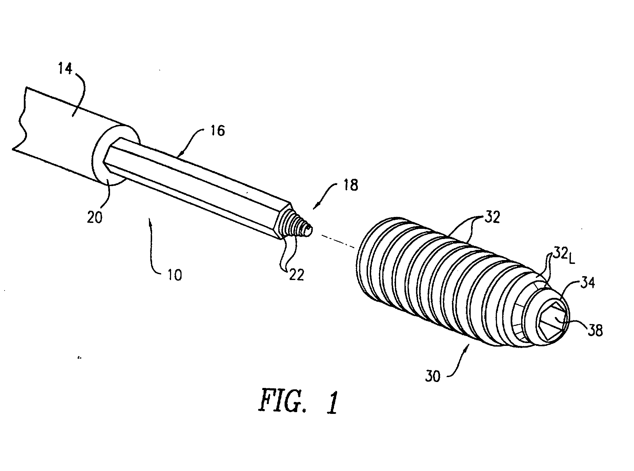

Figure 1 shows a rotatable driver 10 having a tool holder 14 and a tool 16 with a threaded tip 18. A screw abutment surface 20 may be provided on the tool holder 14 at the conjunction of the tool holder 14 and the tool 16. As shown, the tool holder 14 and the tool 16 mat be a monolithic structure, such as a unitary stainless steel forging, or it may be composite with the tool 16 inserting into and interlocking with the tool holder 14 in a conventional manner. The tool holder 14 may be adapted to be grasped manually by virtue of a handle, a wrench or a powered rotating tool (not shown) to rotate the driver 10.

The tool 16 shown has an elongated hexagonal body like a hex wrench, but could be of another shape, such as polygonal, cross, star, or oval shape. Threaded tip 18 is conically tapered and has helical lead-in screw threads 22. The lead-in threads 22 are the means of tapping a previously drilled hole in bone to enable a screw 30 to be more easily threaded into the hole.

The rotatable driver 10 may be used to drive the interference screw 30 into a hole in bone. Interference screw 30 is a longitudinally elongated cylindrical body with external threads 32 that are conically tapered running from approximately the middle of the interference screw 30 to the distal end 34 thereof thereby forming lead-in or starter threads 32_{L}. A cannula 38 extends through the interference screw 30. Threads 32 are like-handed relative to the lead-in threads 22 of the tool 16 such that they follow the lead-in threads 22 into the hole in bone. Threads 32, in conjunction with the wedging effect of screw 30, are the means of fixing the bone blocks in the bone tunnels during an ACL reconstruction procedure. The cannula 38 shown is hexagonal in shape to match the shape of tool 16 shown. One skilled in the art could envision screw cannula 38 being other shapes for receiving a mating tool 16. These include, but are not limited to, polygonal, cross, star, or oval shapes.

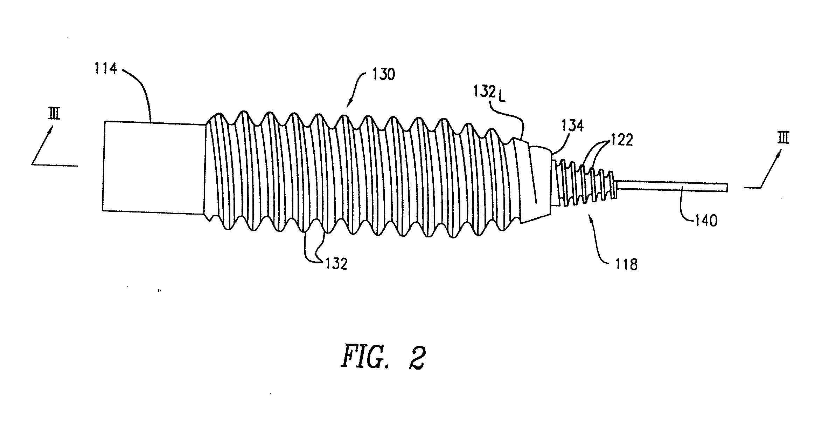

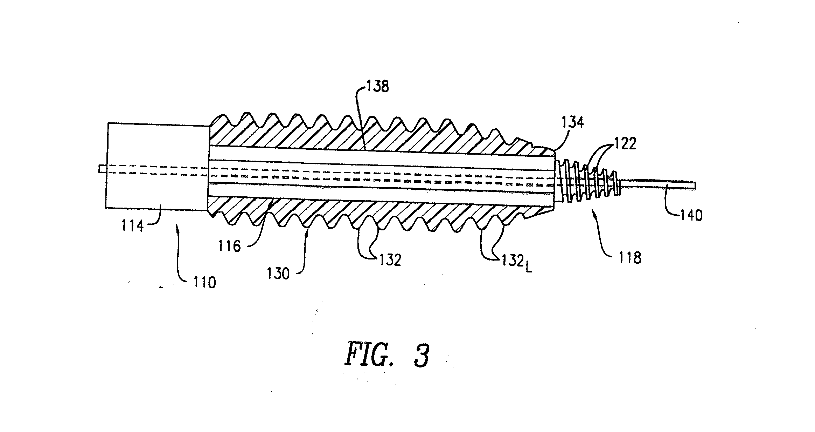

Figures 2 and 3 show an interference screw 130 installed on the tool 116 of the rotatable driver 110. Reference numbers in Figures 2 and 3 are the same as those used in Figure 1 for elements having the same or comparable form and function, but increased by one hundred. The dimensions of tool 116 and cannula 138 are preferably selected such that there is a close fit therebetween and preferably a friction fit. When the screw 130 is pressed against and abutting the tool holder 114, the threaded tip 118 extends beyond the distal end 134 of the screw 130.

Although not shown, lead-in threads 132_{L} may be provided with one or more flutes generally parallel to the axis of the threaded tip 118 as are found on conventional self-threading screws and taps to facilitate thread cutting. The rotatable driver 110 is cannulated to allow passage of a guidewire 140 therethrough. Guidewire 140 allows accurate positioning of the threaded tip 118 during the ACL reconstruction procedure in accordance with known techniques.

The rotatable driver 10,110 of the present invention can be used in the Jones procedure for the reconstruction of the ACL. After the steps of harvesting and preparing the patellar tendon graft, preparing the graft site by drilling a hole through the tibia and femur and placing the graft, the rotatable driver 10, 110 of the present invention is used for turning the screw 30, 130 into the bone tunnel, i.e., between the bone blocks and bone tunnels. Screws 30, 130 may be used to rigidly fix the upper and lower bone blocks in place within the bone tunnel. When the screw 30, 130 is fully threaded into position within the bone, a rearward pull on the driver 10, 110 will allow the tool 16, 116 to be withdrawn from the cannula 38, 138. If the threaded tip 18, 118 is threaded into bone prior to withdrawal, a small amount of bone will be withdrawn with the thread tip 18, 118.

Screws 30, 130 for use with the driver 10, 110 of the present invention may be formed from biocompatable, bioabsorbable materials, such as bioabsorbable polymers, glasses or ceramics, autograft, allograft, or xenograft bone tissues, or combinations of absorbable ceramics, glasses and polymers. In a preferred embodiment, the screw is formed from composites prepared by incorporating bioabsorbable glass or ceramic reinforcements such as fibers or particles in bioabsorbable polymer matrix.

The following examples are illustrative of the principles and practice of this invention, although the present invention is not limited to these specific embodiments. Numerous additional embodiments within the scope and spirit of the invention will be apparent to those skilled in the art.

### Example 1:

Drivers and mating composite screws were fabricated to test the self-threading function of the driver of the present invention. Two drivers were tested. The first was a standard stainless steel hexagonal driver. The second was a self-threading driver, made from stainless steel, that had a leading tip with a self-threading design. The tip extended beyond the screw and incorporated all of the features of the present invention described above in reference to Figure 1. The cannulated screws were composed of a composite of 15/85 (volume percent) β-tricalcium phosphate (TCP) particles (10-micron average diameter) in poly(lactic acid), or PLA polymer. These screws were machined from billets of TCP/PLA composites previously formed by injection molding.

The medial aspect of the medial femoral condyle on a porcine femur was dissected to remove the soft tissue. A hole was drilled with a twist bit into the bone. The hole was positioned to be nearly perpendicular to the bone surface and placed approximately at the insertion point of the medial collateral ligament. The hole measured about two millimeters in diameter.

First, a cannulated composite bone screw mounted on a standard hexagonal driver was used in an attempt to drive the screw into the bone. This proved to be unsuccessful as the leading edge of the screw spun repeatedly and failed to engage the bone. Next, the same type of screw was mounted onto the self-threading driver. In this instance, the self-threading driver tip immediately engaged the bone and advanced the driver/screw complex forward so that the leading edge of the screw gained purchase into the bone. The composite screw was then able to advance into the bone.

### Example 2:

A self-threading driver with a mounted composite screw was made for simulation of a bone-tendon-bone ACL repair. The driver had a leading tip, made from stainless steel with a self-threading design. The tip extended beyond the screw and incorporated all of the features of the present invention described above in reference to Figures 2 and 3. The screw was composed of a composite of 15/85 (volume percent) β-tricalcium phosphate (TCP) particles (10-micron average diameter) in poly(lactic acid), or PLA polymer. The screw was machined from a billet of TCP/PLA composite previously formed by injection molding.

A fresh frozen porcine knee model was used for simulation of a bone-tendon-bone ACL repair. The shaft of the femur was securely clamped in a bone vice. The capsular soft tissue structures of the knee were dissected and removed, exposing the articular surfaces. A patellar bone plug, sized to 11-mm in diameter, was harvested using an oscillating surgical saw. A portion of the patellar tendon was left intact and attached to the bone plug. A bone tunnel was prepared in the femoral condyle by over-drilling a guide pin placed at the insertion of the ACL and driven in an anterio-lateral direction. A 12-mm diameter tunnel was drilled. The patellar bone plug was inserted completely into the tunnel until its distal end intruded two to three millimeters beyond the tunnel opening. Since the bone plug was 11-mm in diameter and the tunnel was 12-mm in diameter, there existed a 1-mm gap into which the screw could be driven. The self-tapping hexagonal driver with the mounted screw was positioned so that during insertion, the threads of the screw would come into contact with the cancellous surface of the bone plug. The driver/screw complex was then advanced by screwing into the tunnel until the back end of the screw was positioned nearly flush with the original cortical surface of the bone. During the insertion process, the composite screw was under torsional resistance. A "biting" sound was heard, indicating that the screw threads were cutting into the bone materials. The bone plug was kept from advancing further into the tunnel during screw insertion by keeping constant tension on the patellar tendon.

In summary, the composite screw was successfully driven into the bone tunnel/bone plug gap. The self-threading driver with mounted composite screw was easily driven into the bone tunnel/bone plug gap once the threads on the stainless steel front component of the driver caught onto the bone.

## Claims

1. A driver for threading a cannulated screw into a substrate, comprising:
a tool with a screw engaging portion and a threaded tip, said screw engaging portion extendable through a cannula formed in the cannula screw, said screw engaging portion having a shape that substantially prevents rotation of said screw engaging portion relative to the cannula screw when said screw engaging portion is extending through the cannula of the cannulated screw, said threaded tip extending beyond the screw when said screw engaging portion is inserted into the cannula of the cannulated screw.

2. The driver of Claim 1, wherein said tip has self-threading features.

3. The driver of Claim 2, wherein said tip tapers down in a direction distal to the screw.

4. The driver of Claim 3, wherein said tip tapers approximately to a point.

5. The driver of Claim 1, further including a screw abutment surface disposed proximate said screw engaging portion distal to said threaded tip, said screw abutment surface extending generally perpendicularly to said screw engaging portion and limiting the axial travel of the screw on said tool engaging portion in a direction distal to said threaded tip.

6. The driver of Claim 5, wherein the cannula of the screw has a friction fit over said tool engaging portion.

7. The driver of Claim 1, wherein said tool engagement portion and the cannula of the screw are both hexagonal in cross-section.

8. A system for fastening orthopedic prosthesis, comprising:
a screw with an axial cannula:
a tool according to any one of Claims 1 to 7 having a screw engaging portion extendable through said cannula.

9. The system of Claim 8, wherein said screw is at least partially non-metallic.

10. The system of Claim 9, wherein said screw has tapered, lead-in threads.

11. The system of Claim 10, wherein said threaded tip is self-threading into bone and wherein the threads of said threaded tip are of the same general direction and pitch as those of the threads on the screw, such that when said screw is placed on said tool engaging portion and said threaded tip is threaded into bone, the threads on said screw follow the threads of said tapered tip into the bone.

12. The system of Claim 8, wherein said screw is made at least partially from a bioabsorbable material.

13. The system of Claim 12, wherein said screw is formed from TCP particles in a PLA polymer.

14. The system of Claim 13, wherein said screw has a friction fit on said screw engaging portion.

15. A driver of any one of Claims 1 to 7 or a system of any one of Claims 8 to 14 for use in introducing cannulated screws into a substrate by:
(A) providing the screw engaging portion with a length greater than that of the screw;
(B) extending the screw engaging portion through the cannulated screw, a tip of the screw engaging portion extending beyond the screw;
(C) providing threads on the tip of the screw engaging portion;
(D) drilling a pilot hole into the substrate;
(E) inserting the tip of the screw engaging portion into the pilot hole;
(F) turning the screw engaging portion and the tip thereof;
(G) threading the threads of the tip into the pilot hole;
(H) continuing to turn the screw engaging portion to draw the tip deeper into the pilot hole and introducing the screw into the pilot hole.
